# EUROPEAN PATENT SPECIFICATION

(11) **EP 3 278 707 B1**
(45) Date of publication and mention of the grant of the patent: **10.07.2019**
(21) Application number: 16771882.4
(22) Date of filing: 03.02.2016
(51) Int. Cl.: A61B 1/00, A61B 1/04, G02B 23/24, G02B 23/26, G06T 1/00

(54) **ENDOSCOPIC DIAGNOSTIC DEVICE, IMAGE PROCESSING METHOD, PROGRAM, AND RECORDING MEDIUM**
ENDOSKOPISCHE DIAGNOSEVORRICHTUNG, BILDVERARBEITUNGSVERFAHREN, PROGRAMM UND AUFZEICHNUNGSMEDIUM
DISPOSITIF DE DIAGNOSTIC ENDOSCOPIQUE, PROCÉDÉ DE TRAITEMENT D'IMAGE, PROGRAMME ET SUPPORT D'ENREGISTREMENT

(30) Priority: 31.03.2015 JP 2015070715
(43) Date of publication of application: 07.02.2018
(73) Proprietor: FUJIFILM Corporation, Tokyo 106-8620 (JP)
(72) Inventor: SAITO, Takaaki, Ashigarakami-gun Kanagawa 258-8538 (JP)
(74) Representative: Jeffrey, Philip Michael
(86) International application number: PCT/JP2016/053120
(87) International publication number: WO 2016/157998

(56) References cited:
- EP-A1- 1 922 979
- EP-A2- 0 403 399
- WO-A1-2013/121610
- JP-A- S61 165 792
- JP-A- 2003 111 722
- JP-A- 2008 194 156
- US-A1- 2014 296 866

## Description

### BACKGROUND OF THE INVENTION

### 1. Field of the Invention

The present invention relates to an endoscopic diagnosis apparatus having a function of measuring the size of a lesion portion or the like in the case of inserting an endoscope into a subject and observing the inside of the subject, and to an image processing method, a program, and a recording medium.

### 2. Description of the Related Art

An endoscopic diagnosis apparatus is used to observe the inside of a subject. In the case of observing the inside of a subject, an endoscope is inserted into a body cavity of the subject, white light, for example, is emitted from a distal end portion of the endoscope onto a region of interest, reflected light thereof is received, and thereby an endoscopic image is captured. The captured endoscopic image is displayed on a display unit and observed by an operator of the endoscopic diagnosis apparatus.

There is a demand for measuring the size of a lesion portion such as a tumor portion for the purpose of, for example, removing a tumor if the tumor is larger than a predetermined size and conserving the tumor for monitoring if the tumor has the predetermined size or less, as well as determining the presence or absence of a lesion portion by viewing an endoscopic image captured inside a subject.

A method for measuring the size of a lesion portion by using a surgical instrument such as measuring forceps is known. In this method, measuring forceps are inserted from a forceps inlet of an endoscope and are protruded from a forceps outlet at a distal end portion of the endoscope. A tip portion of the measuring forceps has scales for measuring size. The tip portion, which is flexible, is pressed against a region of interest so as to bend, and the scales on the tip portion are read to measure the size of a tumor or the like in the region of interest.

The related art documents related to the present invention include JP2011-183000A (hereinafter referred to as PTL 1) and JP2008-245838 A(hereinafter referred to as PTL 2).

PTL 1 relates to an endoscope apparatus. PTL 1 describes ejecting water streams onto a lesion portion from two openings at a distal end portion of an insertion section of an endoscope and determining, on the basis of the distance between the two water streams being equal to the distance between the two openings, whether or not the lesion portion is larger than or equal to a treatment reference value.

PTL 2 relates to a robotic arm system mounted on an endoscope apparatus. PTL 2 describes setting a plurality of measurement points around a lesion portion by using a tip portion of a surgical instrument or the like, and obtaining the size of the lesion portion through computation on the basis of coordinate information on the measurement points

Document US-A-2014/296 866 discloses an endoscopic diagnosis apparatus comprising an imaging unit, a display unit, an input unit, a pixel size calculating unit that calculates an actual size corresponding to one pixel of the endoscopic image on the basis of the imaging size and the information of the actual size, a scale generating unit, a control unit that causes the endoscopic image and the scales to be combined and displayed on the display unit.

### SUMMARY OF THE INVENTION

In this method, however, inserting the measuring forceps into a forceps channel of the endoscope is required only for measuring the size of a lesion portion. This operation is not only time-consuming but also complex and cumbersome. Furthermore, since the measurement is performed by pressing the tip portion of the measuring forceps against a region of interest of a subject so as to bend the tip portion, measurement accuracy is low. In some portions of a subject, it may be difficult to perform measurement, that is, it may be difficult to press the tip portion against the region of interest of the subject.

Furthermore, the endoscope apparatus according to PTL 1 involves an issue that a special endoscope including two openings for ejecting two water streams from the distal end portion of the insertion section is required, and that only this endoscope is capable of measuring the size of a lesion portion.

In addition, the endoscope apparatus according to PTL 2 involves an issue that a robot arm is required to measure the size of a lesion portion, and that it is necessary to set a plurality of measurement points around the lesion portion by operating the complex robot arm.

An object of the present invention is to solve the issues according to the related art and to provide an endoscopic diagnosis apparatus, image processing method, program, and a recording medium that enable easy measurement of the size of a lesion portion or the like based on an endoscopic image captured through a normal operation without a special operation.

To achieve the object, the present invention provides an endoscopic diagnosis apparatus including an imaging unit that has a plurality of pixels and captures an endoscopic image of a subject from a distal end portion of an endoscope; a display unit that displays the endoscopic image; an input unit that receives an instruction to designate a position in the endoscopic image, the instruction being input by an operator; a region detecting unit that detects, from the position in the endoscopic image, a region having a periodic structure of living tissue of the subject in response to the instruction to designate the position; an imaging size calculating unit that calculates, in number of pixels, an imaging size in the endoscopic image equivalent to a period in the periodic structure of the living tissue in the region having the periodic structure of the living tissue; a size information holding unit that holds information of an actual size equivalent to the period in the periodic structure of the living tissue; a pixel size calculating unit that calculates an actual size corresponding to one pixel of the endoscopic image on the basis of the imaging size and the information of the actual size; a scale generating unit that generates scales indicating an actual size of the subject in the endoscopic image on the basis of the actual size corresponding to the one pixel of the endoscopic image; and a control unit that causes the endoscopic image and the scales to be combined and displayed on the display unit.

Preferably, the imaging size calculating unit calculates the imaging size on the basis of a ratio of pixel values of individual pixels in a spectral image having different color components of the endoscopic image.

Preferably, the imaging size calculating unit calculates the imaging size on the basis of a frequency characteristic of a distribution of pixel values of individual pixels within the region having the periodic structure of the living tissue.

Preferably, the frequency characteristic is a power spectrum.

Preferably, the input unit receives an instruction to designate two positions in the endoscopic image, and the region detecting unit detects, as the region having the periodic structure of the living tissue, a region between the two positions in the endoscopic image in response to the instruction to designate the two positions.

Preferably, the imaging size calculating unit calculates a ratio of pixel values of individual pixels in a spectral image having two color components of the endoscopic image, sets a linear region in the region between the two positions, calculates a power spectrum of a ratio of pixel values of individual pixels in the linear region, detects frequency peaks from the power spectrum, and calculates the imaging size in accordance with an interval between the frequency peaks.

Preferably, the imaging size calculating unit calculates an average of intervals between a plurality of the frequency peaks in the linear region and regards the average as the imaging size.

Preferably, the imaging size calculating unit sets a plurality of linear regions in the region between the two positions, calculates, for each linear region, an average of intervals between a plurality of the frequency peaks in the linear region, further calculates an average of averages of intervals between the frequency peaks in the plurality of linear regions, and regards the average of the averages as the imaging size.

Preferably, the input unit further receives an instruction to start detecting the region having the periodic structure of the living tissue and an instruction to finish detecting the region having the periodic structure of the living tissue before and after receiving the instruction to designate the position, respectively, and the region detecting unit starts detecting the region in response to the instruction to start detecting the region and finishes detecting the region in response to the instruction to finish detecting the region.

Preferably, the input unit further receives an instruction to start detecting the region having the periodic structure of the living tissue before receiving the instruction to designate the position, and the region detecting unit starts detecting the region in response to the instruction to start detecting the region and finishes detecting the region after a predetermined time period elapses from when the endoscopic image and the scales are combined and displayed on the display unit.

Preferably, the periodic structure of the living tissue is a microvasculature of a glandular structure of a large intestine, and the period in the periodic structure of the living tissue is an interval between microvessels in the microvasculature of the glandular structure of the large intestine.

Preferably, the periodic structure of the living tissue is a microvasculature in an outermost layer of a mucous membrane of an esophagus, and the period in the periodic structure of the living tissue is an interval between microvessels in the microvasculature in the outermost layer of the mucous membrane of the esophagus.

The present invention also provides an image processing method including a step of holding, with a size information holding unit, information of an actual size equivalent to a period in a periodic structure of living tissue of a subject; a step of causing, with a control unit, an endoscopic image of the subject captured by an imaging unit having a plurality of pixels from a distal end portion of an endoscope to be displayed on a display unit; a step of receiving, with an input unit, an instruction to designate a position in the endoscopic image, the instruction being input by an operator; a step of detecting, with a region detecting unit, a region having the periodic structure of the living tissue from the position in the endoscopic image in response to the instruction to designate the position; a step of calculating in number of pixels, with an imaging size calculating unit, an imaging size in the endoscopic image equivalent to the period in the periodic structure of the living tissue in the region having the periodic structure of the living tissue; a step of calculating, with a pixel size calculating unit, an actual size corresponding to one pixel of the endoscopic image on the basis of the imaging size and the information of the actual size; a step of generating, with a scale generating unit, scales indicating an actual size of the subject in the endoscopic image on the basis of the actual size corresponding to the one pixel of the endoscopic image; and a step of causing, with the control unit, the endoscopic image and the scales to be combined and displayed on the display unit.

Preferably, the imaging size calculating unit calculates the imaging size on the basis of a ratio of pixel values of individual pixels in a spectral image having different color components of the endoscopic image.

Preferably, the imaging size calculating unit calculates the imaging size on the basis of a frequency characteristic of a distribution of pixel values of individual pixels within the region having the periodic structure of the living tissue.

Preferably, the frequency characteristic is a power spectrum.

Preferably, the input unit receives an instruction to designate two positions in the endoscopic image, and the region detecting unit detects, as the region having the periodic structure of the living tissue, a region between the two positions in the endoscopic image in response to the instruction to designate the two positions.

Preferably, the imaging size calculating unit calculates a ratio of pixel values of individual pixels in a spectral image having two color components of the endoscopic image, sets a linear region in the region between the two positions, calculates a power spectrum of a ratio of pixel values of individual pixels in the linear region, detects frequency peaks from the power spectrum, and calculates the imaging size in accordance with an interval between the frequency peaks.

Preferably, the imaging size calculating unit calculates an average of intervals between a plurality of the frequency peaks in the linear region and regards the average as the imaging size.

Preferably, the imaging size calculating unit sets a plurality of linear regions in the region between the two positions, calculates, for each linear region, an average of intervals between a plurality of the frequency peaks in the linear region, further calculates an average of averages of intervals between the frequency peaks in the plurality of linear regions, and regards the average of the averages as the imaging size.

The present invention also provides a program that causes a computer to execute the individual steps of the image processing method described above.

The present invention also provides a computer-readable recording medium on which a program is recorded, the program causing a computer to execute the individual steps of the image processing method described above.

According to the present invention, the size of a lesion portion or the like can be easily measured by using an endoscopic image captured through a normal operation, not by using an endoscopic image captured for the purpose of measuring the size of a lesion portion or the like.

### BRIEF DESCRIPTION OF THE DRAWINGS

Fig. 1 is an external view of an embodiment illustrating the configuration of an endoscopic diagnosis apparatus according to the present invention;
Fig. 2 is a block diagram illustrating the internal configuration of the endoscopic diagnosis apparatus illustrated in Fig. 1;
Fig. 3 is a conceptual diagram illustrating the configuration of a distal end portion of an endoscope;
Fig. 4 is a graph illustrating an emission spectrum of blue laser light emitted by a blue laser light source and of light obtained by converting the wavelength of the blue laser light by using fluorescent bodies;
Fig. 5 is a conceptual diagram illustrating an endoscopic image of a large intestine;
Fig. 6 is a conceptual diagram illustrating a state of a microvasculature of a glandular structure of the large intestine;
Fig. 7 is a conceptual diagram illustrating a state in which a region of the microvasculature is designated;
Fig. 8 is a graph illustrating a power spectrum of a ratio of pixel values of individual pixels in a linear region set in the region of the microvasculature; and
Fig. 9 is a conceptual diagram illustrating an endoscopic image of a microvasculature in an outermost layer of a mucous membrane of an esophagus.

### DESCRIPTION OF THE PREFERRED EMBODIMENTS

Hereinafter, an endoscopic diagnosis apparatus, image processing method, program, and a recording medium according to the present invention will be described in detail on the basis of preferred embodiments illustrated in the attached drawings.

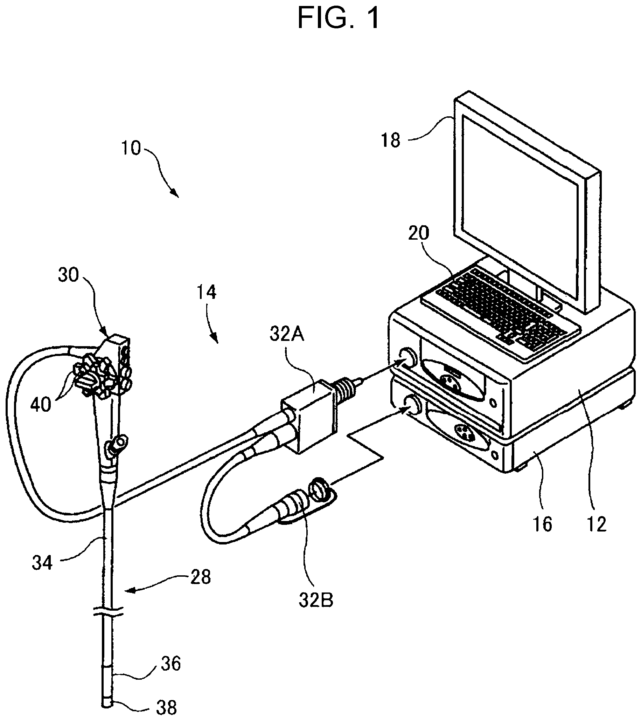

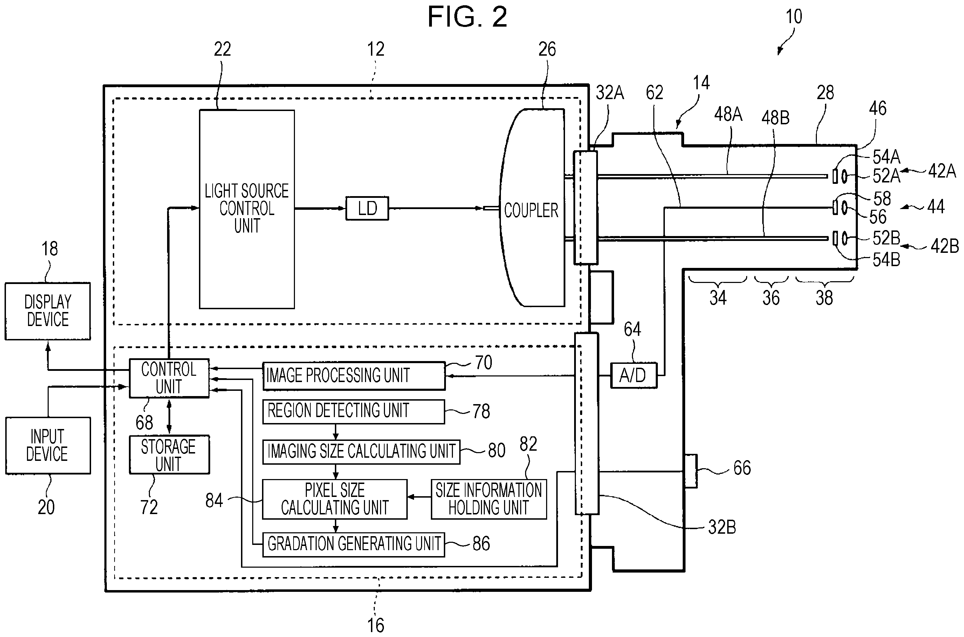

Fig. 1 is an external view of an embodiment illustrating the configuration of the endoscopic diagnosis apparatus according to the present invention, and Fig. 2 is a block diagram illustrating the internal configuration thereof. The endoscopic diagnosis apparatus 10 illustrated in these figures is constituted by a light source device 12, an endoscope 14 that captures an endoscopic image of a region of interest of a subject by using light emitted by the light source device 12, a processor device 16 that performs image processing on the endoscopic image captured by the endoscope 14, a display device 18 that displays the endoscopic image that has undergone the image processing and has been output from the processor device 16, and an input device 20 that receives an input operation.

The light source device 12 is constituted by a light source control unit 22, a laser light source LD, and a coupler (optical splitter) 26.

In this embodiment, the laser light source LD emits narrowband light having a center wavelength of 445 nm in a predetermined blue wavelength range (for example, the center wavelength ± 10 nm). The laser light source LD is a light source that emits, as illumination light, excitation light for causing fluorescent bodies described below to generate white light (pseudo white light). ON/OFF (light-up/light-down) control and light amount control of the laser light source LD are performed by the light source control unit 22, which is controlled by a control unit 68 of the processor device 16 described below.

As the laser light source LD, an InGaN-based laser diode of a broad area type can be used. Alternatively, an InGaNAs-based laser diode, a GaNAs-based laser diode, or the like can be used.

A white light source for generating white light is not limited to a combination of excitation light and fluorescent bodies, and any light source that emits white light may be used. For example, a xenon lamp, halogen lamp, white LED (light-emitting diode), or the like can be used. The wavelength of the laser light emitted by the laser light source LD is not limited to the foregoing example, and laser light with a wavelength that plays a similar role can be selected as appropriate.

The laser light emitted by the laser light source LD enters an optical fiber through a condenser lens (not illustrated) and is then transmitted to a connector section 32A after being split into two branches of light by the coupler 26. The coupler 26 is constituted by a half-mirror, reflection mirror, or the like.

The endoscope 14 is an electronic endoscope having an illumination optical system that emits two branches (two beams) of illumination light from a distal end surface 46 of an endoscope insertion section that is to be inserted into a subject, and an imaging optical system of a single system (single lens) type that captures an endoscopic image of a region of interest. The endoscope 14 includes the endoscope insertion section 28, an operation section 30 that is operated to bend a distal end of the endoscope insertion section 28 or to perform observation, and connector sections 32A and 32B for connecting the endoscope 14 to the light source device 12 and the processor device 16 in a detachable manner.

The endoscope insertion section 28 is constituted by a flexible portion 34 having flexibility, a bending portion 36, and a distal end portion (hereinafter also referred to as an endoscope distal end portion) 38.

The bending portion 36 is disposed between the flexible portion 34 and the distal end portion 38 and is configured to be freely bent by a rotational operation of an angle knob 40 located at the operation section 30. The bending portion 36 can be bent in an arbitrary direction or at an arbitrary angle in accordance with a portion or the like of a subject for which the endoscope 14 is used, and accordingly the endoscope distal end portion 38 can be oriented toward a desired portion of interest.

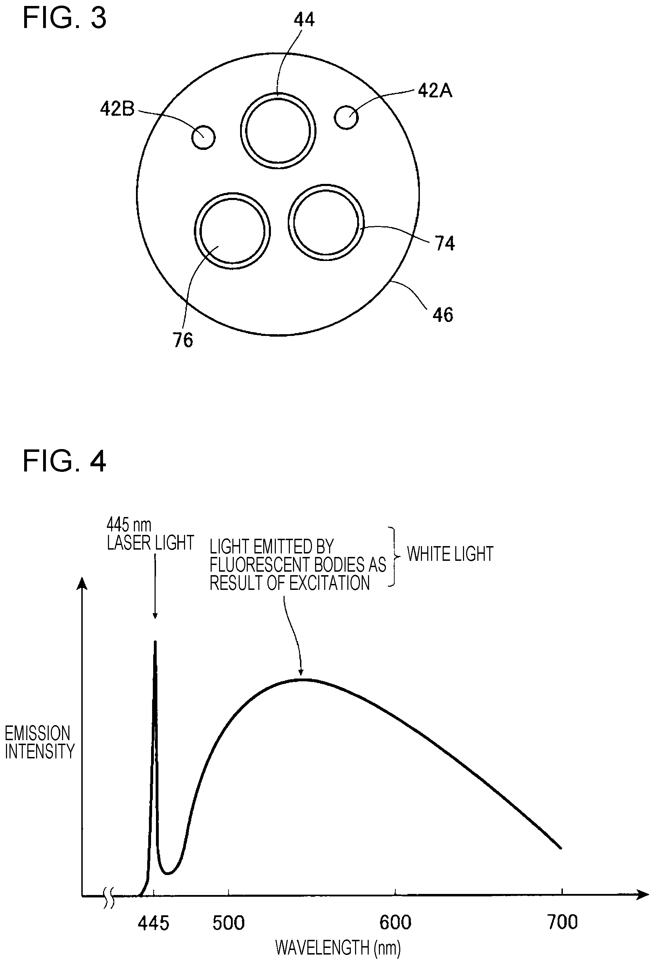

As illustrated in Fig. 3, two illumination windows 42A and 42B for emitting light onto a region of interest, one observation window 44 for gathering reflected light from the region of interest, a forceps outlet 74 serving as an exit for a surgical instrument or the like that is inserted into a forceps channel disposed inside the endoscope insertion section 28, an air/water supply channel opening 76 serving as an exit of an air/water supply channel, and so forth are located on the distal end surface 46 of the endoscope insertion section 28.

The observation window 44, the forceps outlet 74, and the air/water supply channel opening 76 are located in a center portion of the distal end surface 46. The illumination windows 42A and 42B are located on both sides of the observation window 44 so as to sandwich the observation window 44.

An optical fiber 48A is accommodated behind the illumination window 42A. The optical fiber 48A extends from the light source device 12 to the endoscope distal end portion 38 through the connector section 32A. A fluorescent body 54A is located in front of a tip portion (on the illumination window 42A side) of the optical fiber 48A, and in addition an optical system such as a lens 52A is attached in front of the fluorescent body 54A. Likewise, an optical fiber 48B is accommodated behind the illumination window 42B. A fluorescent body 54B and an optical system such as a lens 52B are located in front of a tip portion of the optical fiber 48B.

The fluorescent bodies 54A and 54B contain a plurality of kinds of fluorescent substances (for example, a YAG-based fluorescent substance or a fluorescent substance such as BAM (BaMgAl₁₀O₁₇)) that absorb a part of blue laser light emitted by the laser light source LD and that are excited to emit light in the green to yellow spectrum. When the fluorescent bodies 54A and 54B are irradiated with excitation light, light in the green to yellow spectrum (fluorescent light) emitted by the fluorescent bodies 54A and 54B as a result of excitation is combined with blue laser light that has passed through the fluorescent bodies 54A and 54B without being absorbed, and thereby white light (pseudo white light) for observation is generated.

Fig. 4 is a graph illustrating an emission spectrum of blue laser light emitted by the blue laser light source and of light obtained by converting the wavelength of the blue laser light by using fluorescent bodies. The blue laser light emitted by the laser light source LD is expressed by an emission line having a center wavelength of 445 nm, and the light emitted by the fluorescent bodies 54A and 54B as a result of excitation caused by the blue laser light has a spectral intensity distribution in which the emission intensity increases in a wavelength range of about 450 to 700 nm. Composite light of the light emitted as a result of excitation and the blue laser light forms the foregoing pseudo white light.

The white light according to the present invention is not limited to light strictly including all wavelength components of visible light and may be, for example, light including light in specific wavelength bands, for example, wavelength bands of R (red), G (green), and B (blue) as reference colors, as well as the foregoing pseudo white light. That is, the white light according to the present invention includes, in a broad sense, light including green to red wavelength components, light including blue to green wavelength components, and the like.

In the illumination optical system, the configuration and operation of the illumination window 42A side and the illumination window 42B side are equivalent to each other, and basically equivalent illumination light beams are simultaneously emitted from the illumination windows 42A and 42B. Alternatively, different illumination light beams may be emitted from the illumination windows 42A and 42B. It is not required to have an illumination optical system that emits two branches of illumination light. For example, an equivalent function may be implemented by an illumination optical system that emits one or four branches of illumination light.

An optical system, such as an objective lens unit 56, for gathering image light of a region of interest of a subject is disposed behind the observation window 44. Furthermore, an imaging device 58 (first imaging device), such as a CCD (Charge Coupled Device) image sensor or CMOS (Complementary Metal-Oxide Semiconductor) image senor, for obtaining image information on the region of interest is attached behind the objective lens unit 56. The imaging device 58 corresponds to an imaging unit according to the present invention that has a plurality of pixels and captures an endoscopic image of a subject from the distal end portion of the endoscope 14.

The imaging device 58 receives, at its imaging surface (light receiving surface), light from the objective lens unit 56, photoelectrically converts the received light, and outputs an imaging signal (analog signal). The imaging surface of the imaging device 58 is provided with red (about 580 to 760 nm), green (about 450 to 630 nm), and blue (about 380 to 510 nm) color filters having spectral transmittance for splitting a wavelength range of about 370 to 720 nm of visible light into three bands, and a plurality of sets of pixels, each set formed of pixels of three colors, R pixel, G pixel, and B pixel, are arranged in a matrix on the imaging surface.

The light beams guided by the optical fibers 48A and 48B from the light source device 12 are emitted from the endoscope distal end portion 38 toward a region of interest of a subject. Subsequently, an image depicting a state of the region of interest irradiated with the illumination light is formed on the imaging surface of the imaging device 58 by the objective lens unit 56 and is captured through photoelectric conversion by the imaging device 58. An imaging signal (analog signal) of the captured endoscopic image of the region of interest of the subject is output from the imaging device 58.

The imaging signal (analog signal) of the endoscopic image output from the imaging device 58 is input to an A/D converter 64 through a scope cable 62. The A/D converter 64 converts the imaging signal (analog signal) from the imaging device 58 to an image signal (digital signal). The image signal obtained through the conversion is input to an image processing unit 70 of the processor device 16 through the connector section 32B.

The processor device 16 includes the image processing unit 70, a region detecting unit 78, an imaging size calculating unit 80, a size information holding unit 82, a pixel size calculating unit 84, a scale generating unit 86, the control unit 68, and a storage unit 72. The display device 18 and the input device 20 are connected to the control unit 68. The processor device 16 controls the light source control unit 22 of the light source device 12 and also performs image processing on an image signal of an endoscopic image received from the endoscope 14 and outputs the endoscopic image that has undergone the image processing to the display device 18, in response to an instruction input through an imaging switch 66 of the endoscope 14 or the input device 20.

The display device 18 corresponds to a display unit according to the present invention that displays an endoscopic image. The input device 20 and a button or the like located in the operation section 30 of the endoscope 14 correspond to an input unit according to the present invention that receives various instructions input by an operator.

The image processing unit 70 performs various kinds of image processing, set in advance, on an image signal of an endoscopic image received from the endoscope 14 and outputs an image signal of the endoscopic image that has undergone the image processing. The image signal of the endoscopic image that has undergone the image processing is transmitted to the control unit 68.

The region detecting unit 78 detects, from a position in an endoscopic image corresponding to the image signal of the endoscopic image, a region having a periodic structure of living tissue of a subject in response to an instruction to designate the position in the endoscopic image, which will be described below.

Here, the periodic structure of living tissue is a structure in which specific living tissues are arranged at a constant period in a normal portion of living tissue. There is no periodic structure in a lesion portion of living tissue due to the influence of a lesion, but a normal portion includes a portion in which specific living tissues are arranged at a constant period. For example, microvessels in the microvasculature of a glandular structure of the large intestine or in the microvasculature in an outermost layer of a mucous membrane of the esophagus are arranged at a substantially constant interval (distance) regardless of person, sex, or age.

The periodic structure of living tissue is not limited to the above-described example, and any periodic structure of living tissue may be applied as long as an actual size equivalent to a period in the periodic structure of living tissue is known and as long as the periodic structure is in a region of a normal portion of living tissue that can be imaged together with a region of interest at the time of capturing an endoscopic image.

The imaging size calculating unit 80 calculates, in number of pixels, an imaging size (distance) in the endoscopic image equivalent to a period in the periodic structure of living tissue in the region that has the periodic structure of living tissue and has been detected by the region detecting unit 78.

Here, the imaging size is, in the case of the microvasculature of a glandular structure of the large intestine or the microvasculature in an outermost layer of a mucous membrane of the esophagus, the number of pixels in the endoscopic image corresponding to an interval between microvessels in the microvasculature.

A method for calculating, by using the imaging size calculating unit 80, an imaging size in the endoscopic image equivalent to a period in the periodic structure of living tissue in the region having the periodic structure of living tissue is not limited. For example, the imaging size can be calculated on the basis of the ratio of pixel values of individual pixels in a spectral image having different color components of the endoscopic image or a frequency characteristic of a distribution of pixel values of individual pixels in the region having the periodic structure of living tissue, for example, a power spectrum or the like.

The size information holding unit 82 holds information of an actual size (distance) equivalent to a period in the periodic structure of living tissue.

Here, the actual size equivalent to the period in the periodic structure of living tissue is, in the case of the microvasculature of a glandular structure of the large intestine or the microvasculature in an outermost layer of a mucous membrane of the esophagus, an actual size equivalent to an interval between microvessels in the microvasculature.

The pixel size calculating unit 84 calculates an actual size (distance) corresponding to one pixel of the endoscopic image on the basis of the imaging size calculated by the imaging size calculating unit 80 in number of pixels and the information of the actual size held by the size information holding unit 82.

For example, if the imaging size is X pixels and the actual size is Y mm, the actual size corresponding to one pixel of the endoscopic image can be calculated as Y/X.

The scale generating unit 86 generates scales, such as a scale bar, indicating the actual size of the subject in the endoscopic image, on the basis of the actual size corresponding to one pixel of the endoscopic image calculated by the pixel size calculating unit 84.

The control unit 68 causes the display device 18 to display the endoscopic image that has undergone image processing. In this case, the endoscopic image and the scales generated by the scale generating unit 86 can be combined and displayed on the display device 18 under control of the control unit 68. In addition, the control unit 68 controls the operation of the light source control unit 22 of the light source device 12 and causes, for example, the storage unit 72 to store endoscopic images in units of images (in units of frames) in response to an instruction from the imaging switch 66 of the endoscope 14 or the input device 20.

Next, a description will be given of an operation of the endoscopic diagnosis apparatus 10.

First, a description will be given of an operation in the case of capturing an endoscopic image.

At the time of capturing an endoscopic image, the laser light source LD is lit up with a constant amount of light set in advance under control of the light source control unit 22. Laser light having a center wavelength of 445 nm and emitted by the laser light source LD is applied onto the fluorescent bodies 54A and 54B, and white light is emitted by the fluorescent bodies 54A and 54B. The white light emitted by the fluorescent bodies 54A and 54B is applied onto a subject, the reflected light thereof is received by the imaging device 58, and thereby an endoscopic image of a region of interest of the subject is captured.

An imaging signal (analog signal) of the endoscopic image output from the imaging device 58 is converted to an image signal (digital signal) by the A/D converter 64, various kinds of image processing are performed by the image processing unit 70, and the image signal of the endoscopic image that has undergone the image processing is output. Subsequently, the control unit 68 causes the display device 18 to display an endoscopic image corresponding to the image signal of the endoscopic image that has undergone the image processing, and if necessary, causes the storage unit 72 to store the image signal of the endoscopic image.

Next, a description will be given of an operation in the case of measuring the actual size of a region of interest of a subject.

First, a description will be given of, as a first embodiment, the case of measuring the actual size of a subject in an endoscopic image by using a microvasculature of a glandular structure of a large intestine.

First, an operator of the endoscopic diagnosis apparatus 10 inputs through the input device 20 information of an actual size equivalent to an interval between microvessels in a microvasculature of a glandular structure of a large intestine. The information of the actual size equivalent to the interval between the microvessels is held by the size information holding unit 82.

Subsequently, the operator inserts the endoscope 14 into a subject and moves the distal end portion of the endoscope 14 to a region of interest of the large intestine while checking an endoscopic image displayed on the display device 18.

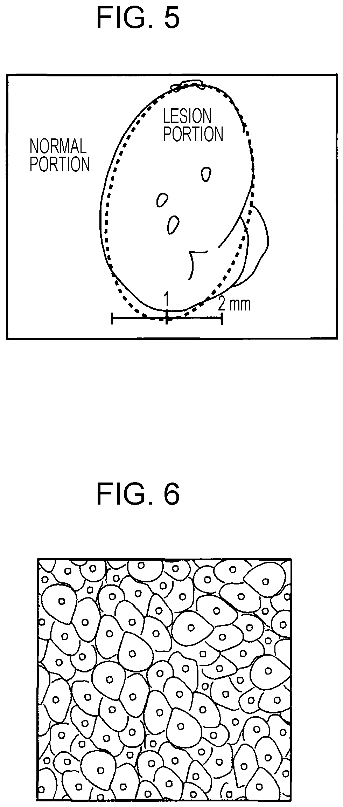

If a lesion portion such as a tumor portion is found in the region of interest during observation of the large intestine, for example, as encompassed by a dotted line in Fig. 5, the operator performs observation such that the microvasculature of the glandular structure of the large intestine in the lesion portion and a normal portion around the lesion portion is included in the endoscopic image. As illustrated in Fig. 6, the microvessels in the microvasculature of the glandular structure of the large intestine in the normal portion are arranged at an interval of 20 to 30 µm.

After moving the distal end portion of the endoscope 14 to the region of interest, the operator presses a button or the like located in the operation section 30 of the endoscope 14 to input an instruction to start detecting a region having a periodic structure of living tissue.

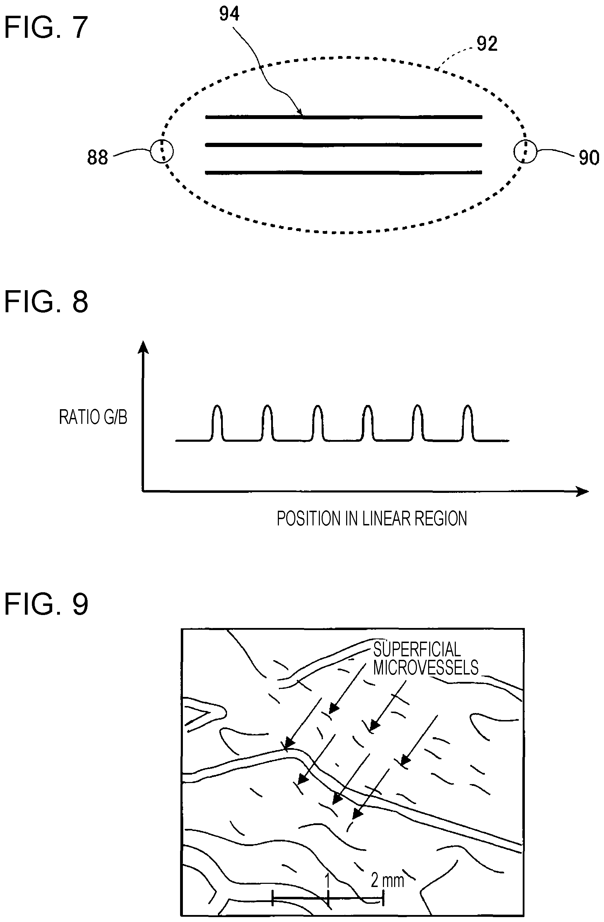

After inputting the instruction to start detecting the region, the operator further inputs through the input device 20 an instruction to designate two positions 88 and 90 that sandwich the microvasculature of the glandular structure of the large intestine in the endoscopic image displayed on the display device 18 as illustrated in Fig. 7, so as to designate the region of the microvasculature of the glandular structure of the large intestine.

Upon input of the instruction to designate the two positions 88 and 90 in the endoscopic image, the region detecting unit 78 starts detecting the region of the microvasculature of the glandular structure of the large intestine from the two positions 88 and 90 in the endoscopic image.

In this case, the region detecting unit 78 detects, as the region of the microvasculature of the glandular structure of the large intestine, a region 92 that is between the two positions in the endoscopic image and encompassed by a dotted line in Fig. 7 in response to the instruction to designate the two positions 88 and 90.

Subsequently, the imaging size calculating unit 80 calculates, in number of pixels, an imaging size in the endoscopic image equivalent to an interval between microvessels in the microvasculature in the region of the microvasculature of the glandular structure of the large intestine detected by the region detecting unit 78.

The imaging size calculating unit 80 is capable of calculating the imaging size in the endoscopic image equivalent to the interval between the microvessels in the microvasculature of the glandular structure of the large intestine in the following manner, for example.

First, the imaging size calculating unit 80 calculates the ratio of pixel values of individual pixels in a spectral image having two color components of the endoscopic image.

For example, the imaging size calculating unit 80 calculates the ratio G/B of pixel values of individual pixels in a spectral image having G (green) and B (blue) components of the endoscopic image. Accordingly, a characteristic structure of living tissue, in the case of this embodiment, the microvasculature of the glandular structure of the large intestine, can be made stand out and extracted.

The spectral image is not limited to a spectral image having individual color components of a white light image that is captured by using white light, and a special optical image that is captured by using special light such as short-wavelength laser light of BLI (Blue Laser Imaging) can also be used.

Subsequently, as illustrated in Fig. 7, the imaging size calculating unit 80 sets a linear region 94 in the region 92 that is between the two positions 88 and 90 in the endoscopic image and has been detected by the region detecting unit 78, that is, in the region of the microvasculature of the glandular structure of the large intestine, in response to the instruction to designate the two positions 88 and 90 in the endoscopic image.

Subsequently, the imaging size calculating unit 80 calculates the power spectrum of the ratio of pixel values of individual pixels in the linear region 94 set in the region of the microvasculature of the glandular structure of the large intestine.

As illustrated in Fig. 8, in which the lateral axis represents the position in the linear region 94 and the vertical axis represents the ratio G/B of pixel values of individual pixels in a spectral image having two color components, the amount of B component is small and the value of the ratio G/B is large at the position of a blood vessel, and thus upward frequency peaks appear at a substantially constant period.

Subsequently, the imaging size calculating unit 80 detects the frequency peaks from the power spectrum and calculates, in accordance with an interval between the frequency peaks, an imaging size in the endoscopic image equivalent to an interval between the microvessels in the microvasculature of the glandular structure of the large intestine.

For example, the imaging size calculating unit 80 is capable of calculating an average of intervals between a plurality of frequency peaks in the linear region 94 and regarding the average as the imaging size in the endoscopic image equivalent to the interval between the microvessels in the microvasculature of the glandular structure of the large intestine. Accordingly, the imaging size can be calculated accurately.

Alternatively, the imaging size calculating unit 80 may set a plurality of linear regions 94 in the region 92 between the two positions 88 and 90, may calculate, for each linear region 94, an average of intervals between a plurality of frequency peaks in the linear region 94, and may further calculate an average of averages of intervals between the frequency peaks in the plurality of linear regions 94. In addition, the imaging size calculating unit 80 may regard the average of the averages as the imaging size in the endoscopic image equivalent to the interval between the microvessels in the microvasculature of the glandular structure of the large intestine. Accordingly, the imaging size can be calculated more accurately.

Subsequently, the pixel size calculating unit 84 calculates an actual size corresponding to one pixel of the endoscopic image, on the basis of the imaging size equivalent to the interval between the microvessels in the microvasculature of the glandular structure of the large intestine calculated in number of pixels by the imaging size calculating unit 80, and on the basis of the information of the actual size equivalent to the interval between the microvessels in the microvasculature of the glandular structure of the large intestine held by the size information holding unit 82.

Subsequently, the scale generating unit 86 generates scales indicating the actual size of the subject in the endoscopic image on the basis of the actual size corresponding to one pixel of the endoscopic image.

Subsequently, under control of the control unit 68, the endoscopic image and the scales are combined and displayed on the display device 18. As the scales, for example, a scale bar in which the length of 1 mm can be seen is displayed on the screen of the display device 18 as illustrated in Fig. 5.

Subsequently, the operator presses a button or the like located in the operation section 30 of the endoscope 14 to input an instruction to finish detecting the region having the periodic structure of living tissue to the endoscopic diagnosis apparatus 10.

Upon input of the instruction to finish detecting the region, the region detecting unit 78 finishes detecting the region of the microvasculature of the glandular structure of the large intestine from the two positions in the endoscopic image. Accordingly, the scales displayed on the display device 18 disappear.

Instead of the finish instruction being input, the region detecting unit 78 may finish detecting the region of the microvasculature of the glandular structure of the large intestine from the two positions in the endoscopic image after a predetermined time period elapses from when the endoscopic image and the scales are combined and displayed on the display device 18.

Next, a description will be given of, as a second embodiment, the case of measuring the actual size of a subject in an endoscopic image by using a microvasculature in an outermost layer of a mucous membrane of an esophagus.

First, as in the case of the first embodiment, an operator inputs through the input device 20 information of an actual size equivalent to an interval between microvessels in a microvasculature in an outermost layer of a mucous membrane of an esophagus, and the information is held by the size information holding unit 82.

Subsequently, the operator inserts the endoscope 14 into a subject and moves the distal end portion of the endoscope 14 to a region of interest of the esophagus while checking an endoscopic image displayed on the display device 18.

Here, if a lesion portion such as a tumor portion is found in the region of interest during observation of the esophagus, the operator performs observation such that the microvasculature in the outermost layer of the mucous membrane of the esophagus in the lesion portion and a normal portion around the lesion portion is included in the endoscopic image. As illustrated in Fig. 9, the microvessels in the microvasculature in the outermost layer of the mucous membrane of the esophagus in the normal portion are arranged at an interval of 100 to 200 µm.

After moving the distal end portion of the endoscope 14 to the region of interest, the operator presses a button or the like located in the operation section 30 of the endoscope 14 to input an instruction to start detecting a region having a periodic structure of living tissue.

After inputting the instruction to start detecting the region, the operator further inputs through the input device 20 an instruction to designate the two positions 88 and 90 that sandwich the microvasculature in the outermost layer of the mucous membrane of the esophagus in the endoscopic image displayed on the display device 18 as illustrated in Fig. 7, so as to designate the region of the microvasculature in the outermost layer of the mucous membrane of the esophagus.

Upon input of the instruction to designate the two positions 88 and 90 in the endoscopic image, the region detecting unit 78 starts detecting the region of the microvasculature in the outermost layer of the mucous membrane of the esophagus from the two positions 88 and 90 in the endoscopic image.

In this case, the region detecting unit 78 detects, as the region of the microvasculature in the outermost layer of the mucous membrane of the esophagus, the region 92 that is between the two positions in the endoscopic image and encompassed by the dotted line in Fig. 7 in response to the instruction to designate the two positions 88 and 90.

Subsequently, the imaging size calculating unit 80 calculates, in number of pixels, an imaging size in the endoscopic image equivalent to an interval between microvessels in the microvasculature in the region of the microvasculature in the outermost layer of the mucous membrane of the esophagus detected by the region detecting unit 78.

Subsequently, the pixel size calculating unit 84 calculates an actual size corresponding to one pixel of the endoscopic image, on the basis of the imaging size equivalent to the interval between the microvessels in the microvasculature in the outermost layer of the mucous membrane of the esophagus calculated in number of pixels by the imaging size calculating unit 80, and on the basis of the information of the actual size equivalent to the interval between the microvessels in the microvasculature in the outermost layer of the mucous membrane of the esophagus held by the size information holding unit 82.

The subsequent operation is similar to that in the case of the first embodiment. Under control of the control unit 68, the endoscopic image and the scales are combined, and a scale bar in which the length of 1 mm can be seen is displayed on the screen of the display device 18, for example, as illustrated in Fig. 9.

In this way, the endoscopic diagnosis apparatus 10 is capable of easily measuring the size of a lesion portion or the like by using an endoscopic image captured through a normal operation, not by using an endoscopic image captured for the purpose of measuring the size of a lesion portion or the like.

In the apparatus according to the present invention, each element included in the apparatus may be constituted by dedicated hardware, or each element may be constituted by a programmed computer.

The method according to the present invention can be implemented by a program that causes a computer to execute individual steps of the method, as described above. Furthermore, a computer-readable recording medium on which the program is recorded can be provided.

The present invention is basically as above.

The present invention has been described in detail above. The present invention is not limited to the above-described embodiments, and various improvements and changes can of course be made without deviating from the gist of the present invention.

### Reference Signs List

- 10: endoscopic diagnosis apparatus
- 12: light source device
- 14: endoscope
- 16: processor device
- 18: display device
- 20: input device
- 22: light source control unit
- 26: coupler (optical splitter)
- 28: endoscope insertion section
- 30: operation section
- 32A, 32B: connector section
- 34: flexible portion
- 36: bending portion
- 38: distal end portion
- 40: angle knob
- 42A, 42B: illumination window
- 44: observation window
- 46: distal end surface
- 48A, 48B: optical fiber
- 52A, 52B: lens
- 54A, 54B: fluorescent body
- 56: objective lens unit
- 58: imaging device
- 62: scope cable
- 64: A/D converter
- 66: imaging switch
- 68: control unit
- 70: image processing unit
- 72: storage unit
- 74: forceps outlet
- 76: air/water supply channel opening
- 78: region detecting unit
- 80: imaging size calculating unit
- 82: size information holding unit
- 84: pixel size calculating unit
- 86: scale generating unit
- 88, 90: position
- 92: region
- 94: linear region
- LD: laser light source

## Claims

1. An endoscopic diagnosis apparatus (10) comprising:
an imaging unit (58) that has a plurality of pixels and captures an endoscopic image of a subject from a distal end portion of an endoscope;
a display unit (18) that displays the endoscopic image;
an input unit (20) that receives an instruction to designate a position in the endoscopic image, the instruction being input by an operator;
a region detecting unit (78) that detects, from the position in the endoscopic image, a region having a periodic structure of living tissue of the subject in response to the instruction to designate the position;
an imaging size calculating unit (80) that calculates, in number of pixels, an imaging size in the endoscopic image equivalent to a period in the periodic structure of the living tissue in the region having the periodic structure of the living tissue;
a size information holding unit (82) that holds information of an actual size equivalent to the period in the periodic structure of the living tissue;
a pixel size calculating unit (84) that calculates an actual size corresponding to one pixel of the endoscopic image on the basis of the imaging size and the information of the actual size;
a scale generating unit (86) that generates scales indicating an actual size of the subject in the endoscopic image on the basis of the actual size corresponding to the one pixel of the endoscopic image; and
a control unit (68) that causes the endoscopic image and the scales to be combined and displayed on the display unit.

2. The endoscopic diagnosis apparatus according to claim 1, wherein
the imaging size calculating unit calculates the imaging size on the basis of a ratio of pixel values of individual pixels in a spectral image having different color components of the endoscopic image.

3. The endoscopic diagnosis apparatus according to claim 1, wherein
the imaging size calculating unit calculates the imaging size on the basis of a frequency characteristic of a distribution of pixel values of individual pixels within the region having the periodic structure of the living tissue.

4. The endoscopic diagnosis apparatus according to claim 3, wherein
the frequency characteristic is a power spectrum.

5. The endoscopic diagnosis apparatus according to any one of claims 1 to 4, wherein
the input unit receives an instruction to designate two positions in the endoscopic image, and
the region detecting unit detects, as the region having the periodic structure of the living tissue, a region between the two positions in the endoscopic image in response to the instruction to designate the two positions.

6. The endoscopic diagnosis apparatus according to claim 5, wherein
the imaging size calculating unit calculates a ratio of pixel values of individual pixels in a spectral image having two color components of the endoscopic image, sets a linear region in the region between the two positions, calculates a power spectrum of a ratio of pixel values of individual pixels in the linear region, detects frequency peaks from the power spectrum, and calculates the imaging size in accordance with an interval between the frequency peaks.

7. The endoscopic diagnosis apparatus according to claim 6, wherein
the imaging size calculating unit calculates an average of intervals between a plurality of the frequency peaks in the linear region and regards the average as the imaging size.

8. The endoscopic diagnosis apparatus according to claim 6, wherein
the imaging size calculating unit sets a plurality of linear regions in the region between the two positions, calculates, for each linear region, an average of intervals between a plurality of the frequency peaks in the linear region, further calculates an average of averages of intervals between the frequency peaks in the plurality of linear regions, and regards the average of the averages as the imaging size.

9. The endoscopic diagnosis apparatus according to any one of claims 1 to 8, wherein
the input unit further receives an instruction to start detecting the region having the periodic structure of the living tissue and an instruction to finish detecting the region having the periodic structure of the living tissue before and after receiving the instruction to designate the position, respectively, and
the region detecting unit starts detecting the region in response to the instruction to start detecting the region and finishes detecting the region in response to the instruction to finish detecting the region.

10. The endoscopic diagnosis apparatus according to any one of claims 1 to 8, wherein
the input unit further receives an instruction to start detecting the region having the periodic structure of the living tissue before receiving the instruction to designate the position, and
the region detecting unit starts detecting the region in response to the instruction to start detecting the region and finishes detecting the region after a predetermined time period elapses from when the endoscopic image and the scales are combined and displayed on the display unit.

11. The endoscopic diagnosis apparatus according to any one of claims 1 to 10, wherein the periodic structure of the living tissue is a microvasculature of a glandular structure of a large intestine, and the period in the periodic structure of the living tissue is an interval between microvessels in the microvasculature of the glandular structure of the large intestine.

12. The endoscopic diagnosis apparatus according to any one of claims 1 to 10, wherein the periodic structure of the living tissue is a microvasculature in an outermost layer of a mucous membrane of an esophagus, and the period in the periodic structure of the living tissue is an interval between microvessels in the microvasculature in the outermost layer of the mucous membrane of the esophagus.

13. An image processing method comprising:
a step of holding, with a size information holding unit, information of an actual size equivalent to a period in a periodic structure of living tissue of a subject;
a step of causing, with a control unit, an endoscopic image of the subject captured by an imaging unit having a plurality of pixels from a distal end portion of an endoscope to be displayed on a display unit;
a step of receiving, with an input unit, an instruction to designate a position in the endoscopic image, the instruction being input by an operator;
a step of detecting, with a region detecting unit, a region having the periodic structure of the living tissue from the position in the endoscopic image in response to the instruction to designate the position;
a step of calculating in number of pixels, with an imaging size calculating unit, an imaging size in the endoscopic image equivalent to the period in the periodic structure of the living tissue in the region having the periodic structure of the living tissue;
a step of calculating, with a pixel size calculating unit, an actual size corresponding to one pixel of the endoscopic image on the basis of the imaging size and the information of the actual size;
a step of generating, with a scale generating unit, scales indicating an actual size of the subject in the endoscopic image on the basis of the actual size corresponding to the one pixel of the endoscopic image; and
a step of causing, with the control unit, the endoscopic image and the scales to be combined and displayed on the display unit.

14. The image processing method according to claim 13, wherein
the imaging size calculating unit calculates the imaging size on the basis of a ratio of pixel values of individual pixels in a spectral image having different color components of the endoscopic image.

15. The image processing method according to claim 13, wherein
the imaging size calculating unit calculates the imaging size on the basis of a frequency characteristic of a distribution of pixel values of individual pixels within the region having the periodic structure of the living tissue.

16. The image processing method according to claim 15, wherein
the frequency characteristic is a power spectrum.

17. The image processing method according to any one of claims 13 to 16, wherein
the input unit receives an instruction to designate two positions in the endoscopic image, and
the region detecting unit detects, as the region having the periodic structure of the living tissue, a region between the two positions in the endoscopic image in response to the instruction to designate the two positions.

18. The image processing method according to claim 17, wherein
the imaging size calculating unit calculates a ratio of pixel values of individual pixels in a spectral image having two color components of the endoscopic image, sets a linear region in the region between the two positions, calculates a power spectrum of a ratio of pixel values of individual pixels in the linear region, detects frequency peaks from the power spectrum, and calculates the imaging size in accordance with an interval between the frequency peaks.

19. The image processing method according to claim 18, wherein
the imaging size calculating unit calculates an average of intervals between a plurality of the frequency peaks in the linear region and regards the average as the imaging size.

20. The image processing method according to claim 18, wherein
the imaging size calculating unit sets a plurality of linear regions in the region between the two positions, calculates, for each linear region, an average of intervals between a plurality of the frequency peaks in the linear region, further calculates an average of averages of intervals between the frequency peaks in the plurality of linear regions, and regards the average of the averages as the imaging size.

21. A program that causes a computer to execute the individual steps of the image processing method according to any one of claims 13 to 20.

22. A computer-readable recording medium on which a program is recorded, the program causing a computer to execute the individual steps of the image processing method according to any one of claims 13 to 20.

## Patentansprüche

1. Endoskopische Diagnosevorrichtung (10), umfassend:
eine Bildgebungseinheit (58), die eine Vielzahl von Pixeln aufweist und ein endoskopisches Bild eines Subjekts von einem distalen Endabschnitt eines Endoskops erfasst;
eine Anzeigeeinheit (18), die das endoskopische Bild anzeigt;
eine Eingabeeinheit (20), die eine Anweisung zur Bestimmung einer Position in dem endoskopischen Bild empfängt, wobei die Anweisung durch einen Bediener eingegeben wird;
eine Regiondetektierungseinheit (78), die aus der Position in dem endoskopischen Bild eine Region mit einer periodischen Struktur von lebendem Gewebe des Subjekts in Reaktion auf die Anweisung zur Bestimmung der Position detektiert;
eine Bildgebungsgrößenberechnungseinheit (80), die als Anzahl von Pixeln eine Bildgebungsgröße in dem endoskopischen Bild berechnet, die zu einer Periode in der periodischen Struktur des lebenden Gewebes in der Region mit der periodischen Struktur des lebenden Gewebes äquivalent ist;
eine Größeninformationsspeichereinheit (82), die Informationen über eine tatsächliche Größe speichert, die zu der Periode in der periodischen Struktur des lebenden Gewebes äquivalent ist;
eine Pixelgrößenberechnungseinheit (84), die auf der Basis der Bildgebungsgröße und der Informationen über die tatsächliche Größe eine tatsächliche Größe berechnet, die einem Pixel des endoskopischen Bildes entspricht;
eine Skalenerzeugungseinheit (86), die auf der Basis der tatsächliche Größe, die dem einen Pixel des endoskopischen Bildes entspricht, Skalen erzeugt, die eine tatsächliche Größe des Subjekts in dem endoskopischen Bild angeben; und
eine Steuerungseinheit (68), die bewirkt, dass das endoskopische Bild und die Skalen kombiniert und auf der Anzeigeeinheit angezeigt werden.

2. Endoskopische Diagnosevorrichtung nach Anspruch 1, wobei
die Bildgebungsgrößenberechnungseinheit die Bildgebungsgröße auf der Basis eines Verhältnisses von Pixelwerten individueller Pixel in einem Spektralbild mit verschiedenen Farbkomponenten des endoskopischen Bildes berechnet.

3. Endoskopische Diagnosevorrichtung nach Anspruch 1, wobei
die Bildgebungsgrößenberechnungseinheit die Bildgebungsgröße auf der Basis eines Frequenzmerkmals einer Verteilung von Pixelwerten individueller Pixel innerhalb der Region mit der periodischen Struktur des lebenden Gewebes berechnet.

4. Endoskopische Diagnosevorrichtung nach Anspruch 3, wobei
das Frequenzmerkmal ein Leistungsspektrum ist.

5. Endoskopische Diagnosevorrichtung nach einem der Ansprüche 1 bis 4, wobei
die Eingabeeinheit eine Anweisung zur Bestimmung von zwei Positionen in dem endoskopischen Bild empfängt und
die Regiondetektierungseinheit als die Region mit der periodischen Struktur des lebenden Gewebes eine Region zwischen den zwei Positionen in dem endoskopischen Bild in Reaktion auf die Anweisung zur Bestimmung der zwei Positionen detektiert.

6. Endoskopische Diagnosevorrichtung nach Anspruch 5, wobei
die Bildgebungsgrößenberechnungseinheit ein Verhältnis von Pixelwerten individueller Pixel in einem Spektralbild mit zwei Farbkomponenten des endoskopischen Bildes berechnet, eine lineare Region in der Region zwischen den zwei Positionen festlegt, ein Leistungsspektrum eines Verhältnisses von Pixelwerten individueller Pixel in der linearen Region berechnet, Frequenzpeaks aus dem Leistungsspektrum detektiert und die Bildgebungsgröße gemäß einem Abstand zwischen den Frequenzpeaks berechnet.

7. Endoskopische Diagnosevorrichtung nach Anspruch 6, wobei
die Bildgebungsgrößenberechnungseinheit einen Durchschnitt von Abständen zwischen einer Vielzahl der Frequenzpeaks in der linearen Region berechnet und den Durchschnitt als die Bildgebungsgröße betrachtet.

8. Endoskopische Diagnosevorrichtung nach Anspruch 6, wobei
die Bildgebungsgrößenberechnungseinheit eine Vielzahl linearer Regionen in der Region zwischen den zwei Position festlegt, für jede lineare Region einen Durchschnitt von Abständen zwischen einer Vielzahl der Frequenzpeaks in der linearen Region berechnet, weiter einen Durchschnitt von Durchschnitten von Abständen zwischen den Frequenzpeaks in der Vielzahl linearer Regionen berechnet und den Durchschnitt der Durchschnitte als die Bildgebungsgröße betrachtet.

9. Endoskopische Diagnosevorrichtung nach einem der Ansprüche 1 bis 8, wobei
die Eingabeeinheit weiter vor und nach dem Empfangen der Anweisung zur Bestimmung der Position eine Anweisung zum Beginn der Detektierung der Region mit der periodischen Struktur des lebenden Gewebes bzw. eine Anweisung zur Beendigung der Detektierung der Region mit der periodischen Struktur des lebenden Gewebes empfängt und
die Regiondetektierungseinheit beginnt, die Region in Reaktion auf die Anweisung zum Beginn der Detektierung der Region zu detektieren, und die Detektierung der Region in Reaktion auf die Anweisung zur Beendigung der Detektierung der Region beendet.

10. Endoskopische Diagnosevorrichtung nach einem der Ansprüche 1 bis 8, wobei
die Eingabeeinheit weiter vor dem Empfangen der Anweisung zur Bestimmung der Position eine Anweisung zum Beginn der Detektierung der Region mit der periodischen Struktur des lebenden Gewebes empfängt und
die Regiondetektierungseinheit beginnt, die Region in Reaktion auf die Anweisung zum Beginn der Detektierung der Region zu detektieren, und die Detektierung der Region beendet, nachdem ein vorgegebener Zeitraum, ab dem das endoskopische Bild und die Skalen kombiniert und auf der Anzeigeeinheit angezeigt werden, verstreicht.

11. Endoskopische Diagnosevorrichtung nach einem der Ansprüche 1 bis 10, wobei die periodische Struktur des lebenden Gewebes ein Mikrogefäßsystem einer Drüsenstruktur eines Dickdarms ist und die Periode in der periodischen Struktur des lebenden Gewebes ein Abstand zwischen Mikrogefäßen in dem Mikrogefäßsystem der Drüsenstruktur des Dickdarms ist.

12. Endoskopische Diagnosevorrichtung nach einem der Ansprüche 1 bis 10, wobei die periodische Struktur des lebenden Gewebes ein Mikrogefäßsystem in einer äußersten Schicht einer Schleimhaut eines Ösophagus ist und die Periode in der periodischen Struktur des lebenden Gewebes ein Abstand zwischen Mikrogefäßen in dem Mikrogefäßsystem in der äußersten Schicht der Schleimhaut des Ösophagus ist.

13. Bildverarbeitungsverfahren, umfassend:
einen Schritt des Haltens, mit einer Größeninformationshaltereinheit, von Informationen über eine tatsächliche Größe, die zu einer Periode in einer periodischen Struktur von lebendem Gewebe eines Subjekts äquivalent ist;
einen Schritt des Bewirkens, mit einer Steuerungseinheit, dass ein endoskopisches Bild des Subjekts, das durch eine Bildgebungseinheit mit einer Vielzahl von Pixeln von einem distalen Endabschnitt eines Endoskops erfasst wird, auf einer Anzeigeeinheit angezeigt wird;
einen Schritt des Empfangens, mit einer Eingabeeinheit, einer Anweisung zur Bestimmung einer Position in dem endoskopischen Bild, wobei die Anweisung durch einen Bediener eingegeben wird;
einen Schritt des Detektierens, mit einer Regiondetektierungseinheit, einer Region mit der periodischen Struktur des lebenden Gewebes aus der Position in dem endoskopischen Bild in Reaktion auf die Anweisung zur Bestimmung der Position;
einen Schritt des Berechnens, als Anzahl von Pixeln, mit einer Bildgebungsgrößenberechnungseinheit, einer Bildgebungsgröße in dem endoskopischen Bild, die zu der Periode in der periodischen Struktur des lebenden Gewebes in der Region mit der periodischen Struktur des lebenden Gewebes äquivalent ist;
einen Schritt des Berechnens, mit einer Pixelgrößenberechnungseinheit, einer tatsächlichen Größe, die einem Pixel des endoskopischen Bildes entspricht, auf der Basis der Bildgebungsgröße und der Informationen über die tatsächliche Größe;
einen Schritt des Erzeugens, mit einer Skalenerzeugungseinheit, von Skalen, die eine tatsächliche Größe des Subjekts in dem endoskopischen Bild angeben, auf der Basis der tatsächlichen Größe, die dem einen Pixel des endoskopischen Bildes entspricht; und
einen Schritt des Bewirkens, mit der Steuerungseinheit, dass das endoskopische Bild und die Skalen kombiniert und auf der Anzeigeeinheit angezeigt werden.

14. Bildverarbeitungsverfahren nach Anspruch 13, wobei
die Bildgebungsgrößenberechnungseinheit die Bildgebungsgröße auf der Basis eines Verhältnisses von Pixelwerten individueller Pixel in einem Spektralbild mit verschiedenen Farbkomponenten des endoskopischen Bildes berechnet.

15. Bildverarbeitungsverfahren nach Anspruch 13, wobei
die Bildgebungsgrößenberechnungseinheit die Bildgebungsgröße auf der Basis eines Frequenzmerkmals einer Verteilung von Pixelwerten individueller Pixel innerhalb der Region mit der periodischen Struktur des lebenden Gewebes berechnet.

16. Bildverarbeitungsverfahren nach Anspruch 15, wobei
das Frequenzmerkmal ein Leistungsspektrum ist.

17. Bildverarbeitungsverfahren nach einem der Ansprüche 13 bis 16, wobei
die Eingabeeinheit eine Anweisung zur Bestimmung von zwei Positionen in dem endoskopischen Bild empfängt und
die Regiondetektierungseinheit als die Region mit der periodischen Struktur des lebenden Gewebes eine Region zwischen den zwei Positionen in dem endoskopischen Bild als Reaktion auf die Anweisung zur Bestimmung der zwei Positionen detektiert.

18. Bildverarbeitungsverfahren nach Anspruch 17, wobei
die Bildgebungsgrößenberechnungseinheit ein Verhältnis von Pixelwerten individueller Pixel in einem Spektralbild mit zwei Farbkomponenten des endoskopischen Bildes berechnet, eine lineare Region in der Region zwischen den zwei Positionen festlegt, ein Leistungsspektrum eines Verhältnisses von Pixelwerten individueller Pixel in der linearen Region berechnet, Frequenzpeaks aus dem Leistungsspektrum detektiert und die Bildgebungsgröße gemäß einem Abstand zwischen den Frequenzpeaks berechnet.

19. Bildverarbeitungsverfahren nach Anspruch 18, wobei
die Bildgebungsgrößenberechnungseinheit einen Durchschnitt von Abständen zwischen einer Vielzahl der Frequenzpeaks in der linearen Region berechnet und den Durchschnitt als die Bildgebungsgröße betrachtet.

20. Bildverarbeitungsverfahren nach Anspruch 18, wobei
die Bildgebungsgrößenberechnungseinheit eine Vielzahl linearer Regionen in der Region zwischen den zwei Position festlegt, für jede lineare Region einen Durchschnitt von Abständen zwischen einer Vielzahl der Frequenzpeaks in der linearen Region berechnet, weiter einen Durchschnitt von Durchschnitten von Abständen zwischen den Frequenzpeaks in der Vielzahl linearer Regionen berechnet und den Durchschnitt der Durchschnitte als die Bildgebungsgröße betrachtet.

21. Programm, das bewirkt, dass ein Computer die individuellen Schritte des Bildverarbeitungsverfahrens nach einem der Ansprüche 13 bis 20 ausführt.

22. Computerlesbares Aufzeichnungsmedium, auf dem ein Programm aufgezeichnet wird, wobei das Programm bewirkt, dass ein Computer die individuellen Schritte des Bildverarbeitungsverfahrens nach einem der Ansprüche 13 bis 20 ausführt.

## Revendications

1. Appareil de diagnostic endoscopique (10) comprenant :
une unité d'imagerie (58) qui comporte une pluralité de pixels et qui capture une image endoscopique d'un sujet à partir d'une portion d'extrémité distale d'un endoscope;
une unité d'affichage (18) qui affiche l'image endoscopique ;
une unité d'entrée (20) qui reçoit une instruction pour désigner une position dans l'image endoscopique, l'instruction étant entrée par un opérateur ;
une unité de détection de région (78) qui détecte, à partir de la position dans l'image endoscopique, une région ayant une structure périodique de tissu vivant du sujet en réponse à l'instruction pour désigner la position ;
une unité de calcul de taille d'imagerie (80) qui calcule, en nombre de pixels, une taille d'imagerie dans l'image endoscopique équivalente à une période dans la structure périodique du tissu vivant dans la région ayant la structure périodique du tissu vivant ;
une unité de maintien d'informations de taille (82) qui maintient des informations d'une taille réelle équivalente à la période dans la structure périodique du tissu vivant ;
une unité de calcul de taille de pixel (84) qui calcule une taille réelle correspondant à un pixel particulier de l'image endoscopique sur la base de la taille d'imagerie et des informations de la taille réelle ;
une unité de création d'échelle (86) qui crée des échelles indiquant une taille réelle du sujet dans l'image endoscopique sur la base de la taille réelle correspondant au pixel particulier de l'image endoscopique ; et
une unité de commande (68) qui amène l'image endoscopique et les échelles à être combinées et affichées sur l'unité d'affichage.

2. Appareil de diagnostic endoscopique selon la revendication 1, dans lequel
l'unité de calcul de taille d'imagerie calcule la taille d'imagerie sur la base d'un rapport de valeurs de pixel de pixels individuels dans une image spectrale ayant des composantes de couleur différentes de l'image endoscopique.

3. Appareil de diagnostic endoscopique selon la revendication 1, dans lequel
l'unité de calcul de taille d'imagerie calcule la taille d'imagerie sur la base d'une caractéristique de fréquence d'une distribution de valeurs de pixel de pixels individuels à l'intérieur de la région ayant la structure périodique du tissu vivant.

4. Appareil de diagnostic endoscopique selon la revendication 3, dans lequel
la caractéristique de fréquence est un spectre de puissance.

5. Appareil de diagnostic endoscopique selon l'une quelconque des revendications 1 à 4, dans lequel
l'unité d'entrée reçoit une instruction pour désigner deux positions dans l'image endoscopique, et
l'unité de détection de région détecte, en tant que région ayant la structure périodique du tissu vivant, une région entre les deux positions dans l'image endoscopique en réponse à l'instruction pour désigner les deux positions.

6. Appareil de diagnostic endoscopique selon la revendication 5, dans lequel
l'unité de calcul de taille d'imagerie calcule un rapport de valeurs de pixel de pixels individuels dans une image spectrale ayant deux composantes de couleur de l'image endoscopique, fixe une région linéaire dans la région entre les deux positions, calcule un spectre de puissance d'un rapport de valeurs de pixel de pixels individuels dans la région linéaire, détecte des pics de fréquence à partir du spectre de puissance, et calcule la taille d'imagerie selon un intervalle entre les pics de fréquence.

7. Appareil de diagnostic endoscopique selon la revendication 6, dans lequel
l'unité de calcul de taille d'imagerie calcule une moyenne d'intervalles entre une pluralité des pics de fréquence dans la région linéaire et considère la moyenne en tant que taille d'imagerie.

8. Appareil de diagnostic endoscopique selon la revendication 6, dans lequel
l'unité de calcul de taille d'imagerie fixe une pluralité de régions linéaires dans la région entre les deux positions, calcule, pour chaque région linéaire, une moyenne d'intervalles entre une pluralité des pics de fréquence dans la région linéaire, calcule en outre une moyenne de moyennes d'intervalles entre les pics de fréquence dans la pluralité de régions linéaires, et considère la moyenne des moyennes en tant que taille d'imagerie.

9. Appareil de diagnostic endoscopique selon l'une quelconque des revendications 1 à 8, dans lequel
l'unité d'entrée reçoit en outre une instruction pour démarrer la détection de la région ayant la structure périodique du tissu vivant et une instruction pour terminer la détection de la région ayant la structure périodique du tissu vivant respectivement avant et après la réception de l'instruction pour désigner la position, et
l'unité de détection de région démarre la détection de la région en réponse à l'instruction de démarrer la détection de la région et termine la détection de la région en réponse à l'instruction de démarrer la détection de la région.

10. Appareil de diagnostic endoscopique selon l'une quelconque des revendications 1 à 8, dans lequel
l'unité d'entrée reçoit en outre une instruction pour démarrer la détection de la région ayant la structure périodique du tissu vivant avant la réception de l'instruction pour désigner la position, et
l'unité de détection de région démarre la détection de la région en réponse à l'instruction de démarrer la détection de la région et termine la détection de la région après qu'une période de temps prédéterminée s'écoule à partir du moment où l'image endoscopique et les échelles sont combinées et affichées sur l'unité d'affichage.

11. Appareil de diagnostic endoscopique selon l'une quelconque des revendications 1 à 10, dans lequel la structure périodique du tissu vivant est un microsystème vasculaire d'une structure glandulaire d'un gros intestin, et la période dans la structure périodique du tissu vivant est un intervalle entre des microvaisseaux dans le microsystème vasculaire de la structure glandulaire du gros intestin.

12. Appareil de diagnostic endoscopique selon l'une quelconque des revendications 1 à 10, dans lequel la structure périodique du tissu vivant est un microsystème vasculaire dans une couche la plus extérieure d'une membrane muqueuse d'un oesophage, et la période dans la structure périodique du tissu vivant est un intervalle entre des microvaisseaux dans le microsystème vasculaire dans la couche la plus extérieure de la membrane muqueuse de l'oesophage.

13. Procédé de traitement d'image comprenant :
une étape de maintien, avec une unité de maintien d'informations de taille, d'informations d'une taille réelle équivalente à une période dans une structure périodique de tissu vivant d'un sujet ;
une étape consistant à amener, avec une unité de commande, une image endoscopique du sujet capturée par une unité d'imagerie ayant une pluralité de pixels provenant d'une portion d'extrémité distale d'un endoscope à être affichée sur une unité d'affichage ;
une étape de réception, avec une unité d'entrée, d'une instruction pour désigner une position dans l'image endoscopique, l'instruction étant entrée par un opérateur ;
une étape de détection, avec une unité de détection de région, d'une région ayant la structure périodique du tissu vivant à partir de la position dans l'image endoscopique en réponse à l'instruction pour désigner la position ;
une étape de calcul en nombre de pixels, avec une unité de calcul de taille d'imagerie, d'une taille d'imagerie dans l'image endoscopique équivalente à la période dans la structure périodique du tissu vivant dans la région ayant la structure périodique du tissu vivant ;
une étape de calcul, avec une unité de calcul de taille de pixel, d'une taille réelle correspondant à un pixel particulier de l'image endoscopique sur la base de la taille d'imagerie et des informations de la taille réelle ;
une étape de création, avec une unité de création d'échelle, d'échelles indiquant une taille réelle du sujet dans l'image endoscopique sur la base de la taille réelle correspondant au pixel particulier de l'image endoscopique ; et
une étape consistant à amener, avec l'unité de commande, l'image endoscopique et les échelles à être combinées et affichées sur l'unité d'affichage.

14. Procédé de traitement d'image selon la revendication 13, dans lequel
l'unité de calcul de taille d'imagerie calcule la taille d'imagerie sur la base d'un rapport de valeurs de pixel de pixels individuels dans une image spectrale ayant des composantes de couleur différentes de l'image endoscopique.

15. Procédé de traitement d'image selon la revendication 13, dans lequel
l'unité de calcul de taille d'imagerie calcule la taille d'imagerie sur la base d'une caractéristique de fréquence d'une distribution de valeurs de pixel de pixels individuels à l'intérieur de la région ayant la structure périodique du tissu vivant.

16. Procédé de traitement d'image selon la revendication 15,
dans lequel la caractéristique de fréquence est un spectre de puissance.

17. Procédé de traitement d'image selon l'une quelconque des revendications 13 à 16, dans lequel
l'unité d'entrée reçoit une instruction pour désigner deux positions dans l'image endoscopique, et
l'unité de détection de région détecte, en tant que région ayant la structure périodique du tissu vivant, une région entre les deux positions dans l'image endoscopique en réponse à l'instruction pour désigner les deux positions.

18. Procédé de traitement d'image selon la revendication 17, dans lequel
l'unité de calcul de taille d'imagerie calcule un rapport de valeurs de pixel de pixels individuels dans une image spectrale ayant deux composantes de couleur de l'image endoscopique, fixe une région linéaire dans la région entre les deux positions, calcule un spectre de puissance d'un rapport de valeurs de pixel de pixels individuels dans la région linéaire, détecte des pics de fréquence à partir du spectre de puissance, et calcule la taille d'imagerie selon un intervalle entre les pics de fréquence.

19. Procédé de traitement d'image selon la revendication 18, dans lequel
l'unité de calcul de taille d'imagerie calcule une moyenne d'intervalles entre une pluralité des pics de fréquence dans la région linéaire et considère la moyenne en tant que taille d'imagerie.

20. Procédé de traitement d'image selon la revendication 18, dans lequel
l'unité de calcul de taille d'imagerie fixe une pluralité de régions linéaires dans la région entre les deux positions, calcule, pour chaque région linéaire, une moyenne d'intervalles entre une pluralité des pics de fréquence dans la région linéaire, calcule en outre une moyenne de moyennes d'intervalles entre les pics de fréquence dans la pluralité de régions linéaires, et considère la moyenne des moyennes en tant que taille d'imagerie.

21. Programme qui amène un ordinateur à exécuter les étapes individuelles du procédé de traitement d'image selon l'une quelconque des revendications 13 à 20.

22. Support d'enregistrement lisible par ordinateur sur lequel un programme est enregistré, le programme amenant un ordinateur à exécuter les étapes individuelles du procédé de traitement d'image selon l'une quelconque des revendications 13 à 20.
